# EUROPEAN PATENT APPLICATION

(11) **EP 2 833 164 A1**
(43) Date of publication of application: **04.02.2015**
(21) Application number: 13179111.3
(22) Date of filing: 02.08.2013
(51) Int. Cl.: G01T 1/169

(54) **Measurement device and method for measuring radiation of a radiation source and controlling device**

(71) Applicant: Koltermann, Peer, 16761 Henningsdorf (DE)
(72) Inventor: Koltermann, Peer, 13595 Berlin (DE)
(74) Representative: Staroske, Sandro

(57) **Abstract**

The invention relates to a measurement device for measuring radiation of a radiation source, comprising a container (2) for receiving a liquid; a detector for detecting radiation of the radiation source, the detector being arranged in the container (2). According to the invention, a mechanism (3) for moving the container (2) to various measurement locations along at least one positioning direction (x, y) is provided, wherein the detector is arranged in the container (2) in such a way that it will be moved along with the container (2) when the container (2) is moved in the positioning direction (x, y).

## Description

The invention relates to a measurement device for measuring radiation of a radiation source according to the preamble of claim 1. Further, the invention relates to a controlling device according to claim 14 and a method for measuring radiation of a radiation source according to claim 15.

In the medical field, the use of radiation for treating malignant tissue is well known. For example, particle radiation or X rays are directed towards the tissue to be treated using, for example, a linear accelerator as radiation source. Such a radiation treatment has to be carried out very accurately both in terms of the impact location of the radiation and the intensity of the radiation at the impact location. Computer system are used for planning these treatments (so-called TPSs - treatment planning systems), wherein a TPS needs accurate data in particular with respect to the intensity of the radiation at the impact location, i.e. at the intended location of the interaction between the radiation and the tissue to be treated.

Therefore, a measurement of the radiation (in particular, of the intensity of the radiation) generated by the radiation source has to be carried out before the radiation treatment. Usually, the radiation measurement is carried using a target having a density similar to those of the human body. For example, a tank filled with water (a so-called "water phantom") is used as target, wherein a detector for detecting the radiation is arranged in the water tank. The detector is moved within the water tank in all three spatial directions x, y, z to a plurality of locations in order to measure the energy (the intensity) of the radiation at various locations in the water tank. In order to be able to detect the whole potential radiation field of the radiation source, a large water tank has to be employed.

It is an object of the invention to facilitate the measurement of the radiation carried out before the treatment or as a routine system check.

According to the invention, a measurement device for measuring radiation of a radiation source is provided, comprising
- a container for receiving a liquid;
- a detector for detecting radiation of the radiation source, the detector being arranged in the container, and
- a mechanism for moving the container to various measurement locations along at least one positioning direction, wherein the detector is arranged in the container in such a way that it will be moved along with the container when the container is moved in the positioning direction.

Instead of using a large container (e.g. the water phantom mentioned above) and moving the detector to various measurement locations, the (complete) container is moved together with the detector. This permits the usage of a considerable smaller container, which, in turn, facilitates the transportation and the set-up of the measurement device. Further, an additional water reservoir required for filling the water tank of a conventional system is not necessary. Rather, the container of the measurement device according to the invention can be manually filled and emptied. Therefore, the whole measurement device according to the invention may fit into a rather small transportation case which can be carried in a car or as normal luggage when using an airplane.

Further, using a smaller container may permit arranging the measurement device on a patient table, wherein the adjustment means (e.g. for adjusting the height) of the patient table can be used for pre-positioning the container. The large water tank of the conventional systems in contrast requires an additional elevating table since the lager water tank is too heavy to be positioned on the patient table.

For example, the measurement device according to the invention is arranged in a transportation case, wherein setting up the measurement device may only consist of arranging the transportation case on the patient table and removing at least a part (such as a cover or a sidewall) of the transportation case. If required, the container can be filled with a liquid of a known volume. It is noted that the measurement device according to the invention can, of course, also be used for a tissue maximum ratio - TMR measurement.

According to an embodiment of the invention, a container having a volume smaller than 50l, smaller than 20l or smaller than 10l is used. For example, the container has a cuboid shape, wherein, however, other geometries might be used. In particular, the dimensions of the container are chosen in such a way that the liquid volume surrounding the detector is sufficient to generate an absorption of the radiation comparable to those of a larger volume container or the human body.

Further, the detector may be arranged in the container in such a way that the detector is movable relative to the container in a direction essentially perpendicular to the positioning direction. This movement of the detector may be realized by a mechanism that includes at least one guide element on which the detector is (directly or indirectly) mounted, wherein the guide element is moved along with the container when the container is moved in the positioning direction. For example, the guide element is arranged stationary relative to the container. The detector may be mounted on the guide element via a detector holder.

The measurement device may be arranged in such a way relative to the radiation source that the positioning direction extends essentially perpendicular to an axis of the radiation emitted by the radiation source. For example, the container comprises a bottom and the positioning direction extends essentially parallel to the bottom of the container.

According to another embodiment of the invention, the mechanism for moving the container is configured for moving the container independently in a first and in a second direction, the second direction running perpendicular to the first direction. For example, the mechanism for moving the container comprises a first and second (e.g. motorized) translation stage arranged perpendicular to one another. For example, the container can be moved in a (horizontal) plane (x-y plane) extending perpendicular to an axis of the radiation emitted by the radiation source and thus to a bottom of the container. Additionally, as set forth above, the detector may be movable relative to the container in a direction parallel to the axis of the radiation (i.e. in the z direction).

For example, the mechanism for moving the container allows the container to be moved to any location of a rectangle (having a, for example, a square size of at least 50 cm x 50 cm or of about 50 cm x 50 cm).

The measurement device according to the invention may also comprise a controlling device (e.g. in the form of a connector box) for controlling at least the mechanism for moving the container and the detector. The controlling device may a particular hardware device, i.e. a device containing circuitry that is adapted to perform the functions of the controlling device. However, it is also conceivable, that the controlling device is realized by a software program running on a computer.

The mechanism for moving the container may be controlled via a first protocol and the detector may be controlled via a different second protocol, wherein the controlling device is configured to communicate via both the first and the second protocol. Thus, the controlling device permits the easy usage of devices (such as the mechanism for moving the container and the detector) of different manufactures. Besides the mechanism for moving the container and the detector, the controlling device may be configured to control (and thus to communicate with) further entities (such as an electrometer and/or an evaluation software for evaluating detector data) of further manufactures, the further entities using, for example, further protocols different from the first and/or the second protocol. Thus, using the controller it may be possible to establish communication between any container, electrometer and/or a software for evaluating detector data and/or for operating the measurement device.

For example, the controlling device comprises a database storing parameters and/or commands of a plurality of protocols.

The invention further relates to a radiation therapy arrangement, comprising a measurement device as claimed in any of the preceding claims and a patient table, wherein the measurement device is arranged on the patient table.

Moreover, the invention relates to a method for measuring radiation of a radiation source, comprising the steps of:
- providing a container for receiving a liquid;
- providing a detector for detecting radiation of the radiation source, the detector being arranged in the container, and
- moving the container in at least one positioning direction, wherein the detector is arranged in the container in such a way that it is moved along with the container in the positioning direction.

In particular, the detector is fixed relative to the container such that, when the container is moved, the detector will move along with the container.

An embodiment of the invention is described hereinafter with reference to the drawings, which show:
- Fig. 1: a perspective view of a measurement device according to an embodiment of the invention;

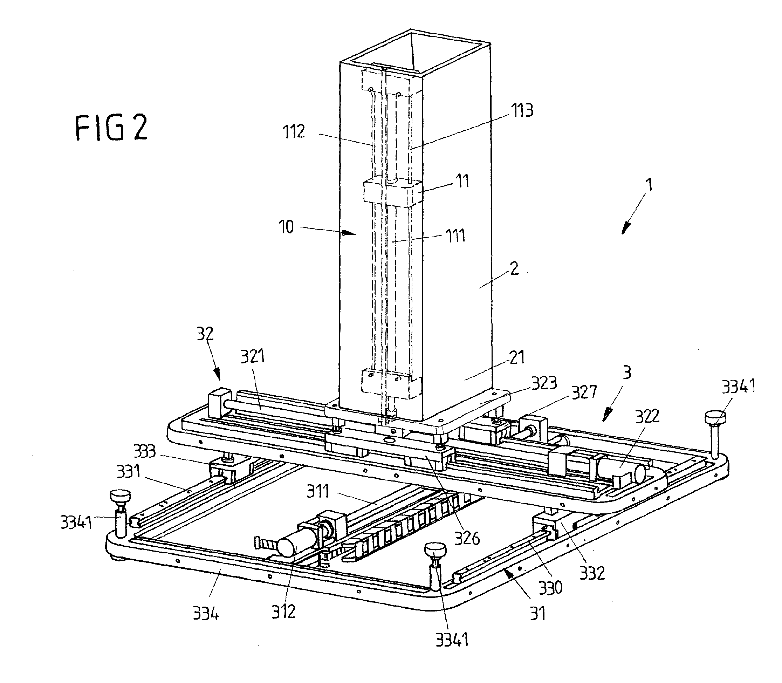

- Fig. 2: a perspective view of the interior of the measurement device of Figure 1;
- Fig. 3: a perspective view of the mechanism for moving the container and the detector of the measurement device of Figures 1 and 2;

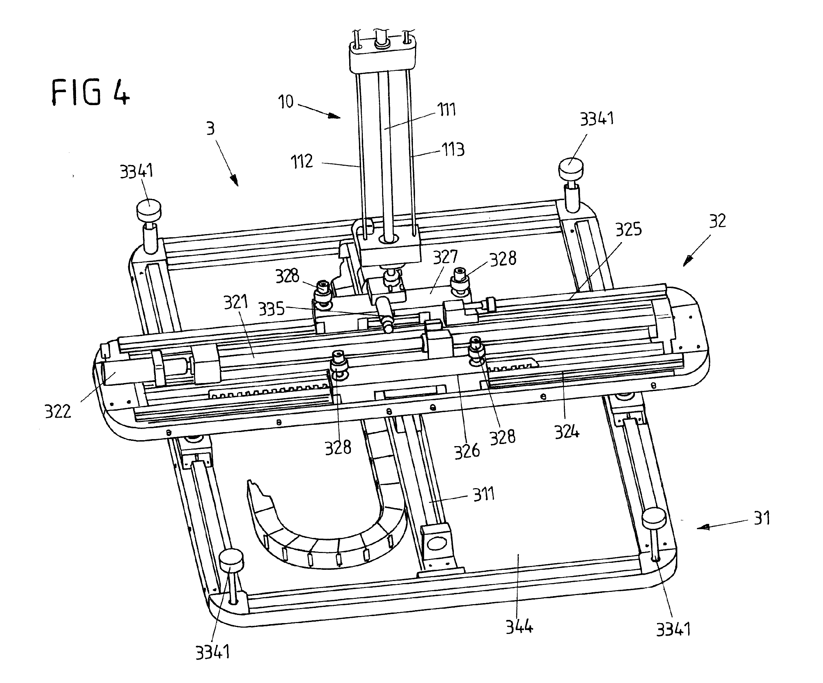

- Fig. 4: another view of the mechanism of Figure 3; and

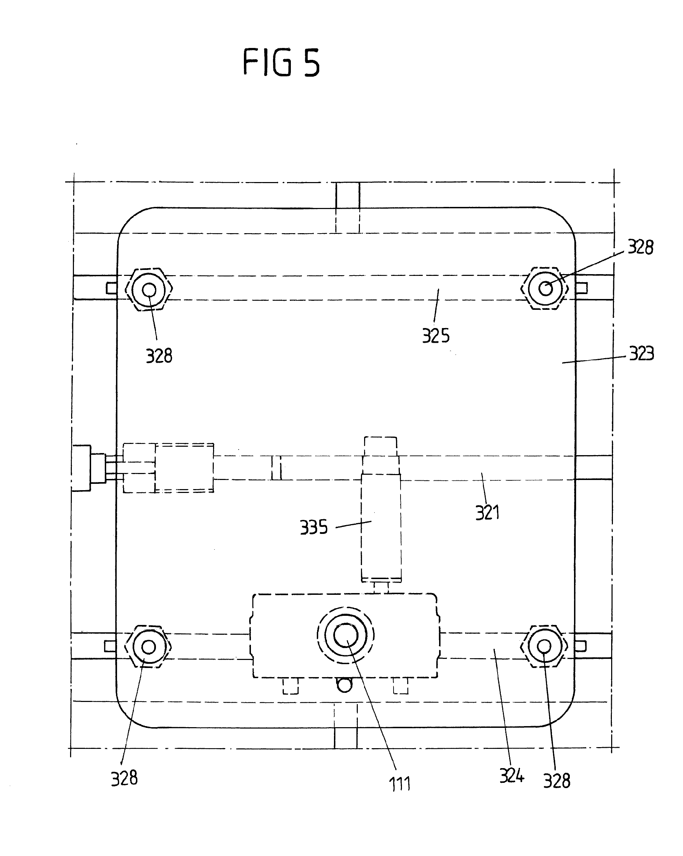

- Fig. 5: a top view of the region of the carrier plate of the measurement device of the preceding Figures.

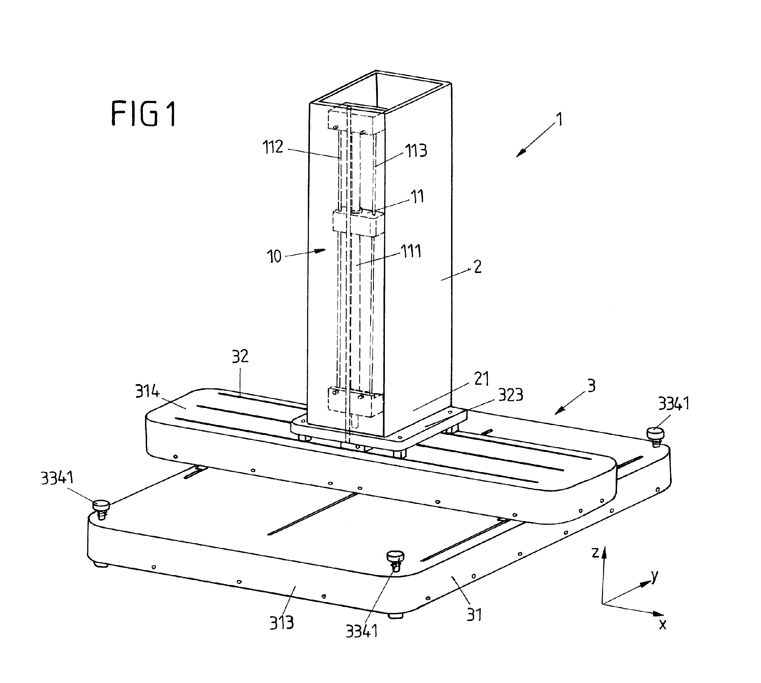

Figure 1 depicts a measurement device 1 for measuring radiation of a radiation source (not shown) according to an embodiment of the invention. The measurement device 1 comprises a container in the form of a tank 2 for receiving a liquid, in particular in the form of water (or any other liquid having a comparable density). The tank 2 may have a volume of not more than 20l.

Further, the measurement device 1 comprises a holder 11 for holding a detector (not shown for detecting radiation of a radiation source (not shown), e.g. an ionization detector or a solid state detector, within the inner volume of tank 2. The measurement device 1 also includes a mechanism 10 for positioning the detector in tank 2 along the z direction, i.e. parallel to an axis of radiation emitted by the radiation source.

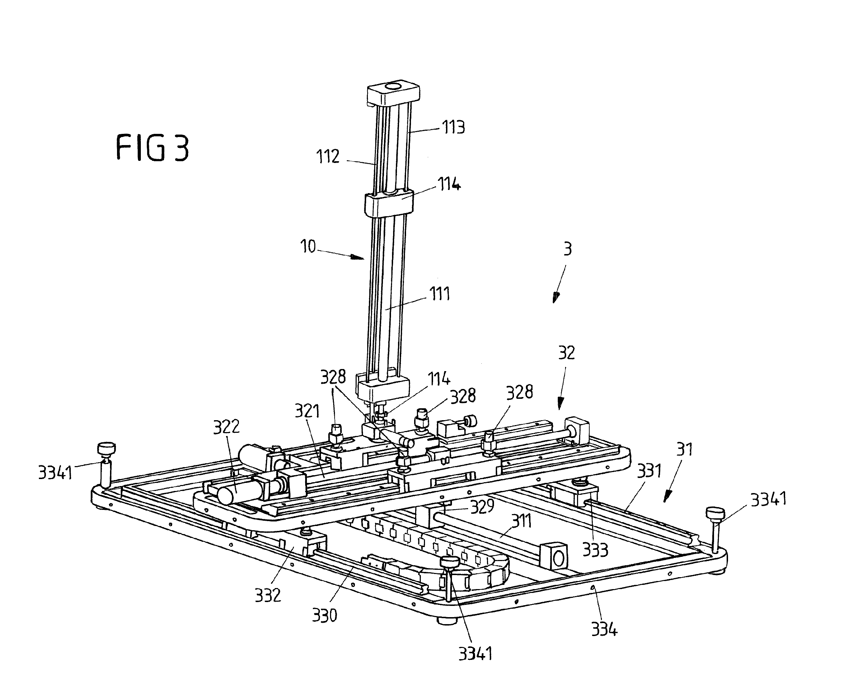

The mechanism 10 for positioning the detector comprises a translation element in the form of a translation shaft 111 interacting with the holder 11 and two guide elements in the form of guide rails 112, 113. By actuating the translation shaft 111 using an electric motor 114 (see e.g. Figure 3) the detector holder 11 and thus the detector can be moved within the tank 2 along the z direction (the vertical direction), i.e. parallel to the axis of radiation emitted by the radiation source. Thus, measurements can be carried out at different locations on the z axis, i.e. at different distances from the radiation source.

Moreover, the measurement device 1 comprises a mechanism 3 for moving the tank 2 independently in a first and a second positioning direction x, y, i.e. for moving the tank 2 in a plane perpendicular to the z direction (perpendicular to the main extension direction of tank 2 or parallel to a bottom 21 of tank 2).

The mechanism 3 comprises a first translation stage 31 for moving tank 2 in the x direction and a second translation stage 32 for moving tank 2 in the y-direction. Each translation stage 31, 32 comprises a translation shaft 311, 321 driven by an actuator 312, 322 such as a stepper motor or a DC-motor. It is noted that the translation stages 31, 32 are, of course, only exemplarily. In principle, any kind of mechanism could be used permitting the tank being moved in a plane perpendicular to the z axis. The translation stages 31, 32 are arranged within cover elements 313, 314 (see Fig. 1).

The tank 2 is arranged on a carrier 323 mounted to the second translation stage 32, wherein the carrier 323 interacts with the translation shaft 321 and is slidably mounted on lateral guide rails 324, 325 via slides 326, 327. The carrier 323 is (e.g. detachably) connected to the slides 326, 327 via adjustment screws 328 permitting the orientation of the carrier 323 and thus of tank 2 to be adjusted. The tank 2, in turn, can be detachably attached to the carrier 323 by attachment means such as screws. However, it is also possible that the tank 2 and the carrier 323 are integrally formed (e.g. from a plastic material). For example, for transportation of the measurement device 1 the unit consisting of the tank 2 and the carrier 323 is detached from the slides 326, 327 (i.e. carrier 323 may remain attached to tank 2).

The second translation stage 32 is mounted to the first translation stage 31 via a guide element 329 interacting with the translation shaft 311 of the first translation stage 31 and two slides 332, 333 slidably arranged on lateral guide rails 330, 331. Using the two translation stages 31, 32 the tank 2 can be moved to any desired measurement position in the x-y plane within the range of the translation stages 31, 32.

Further, the translation shaft 311 and the lateral guide rails 330, 331 of the first translation stage 31 are arranged on a base plate 334. The base plate 334 allows the measurement device 1 to be arranged on a patient table, wherein the orientation of the base plate 334 can be adjusted by adjustment screws 3341.

It is noted that the mechanism 10 for positioning the detector (i.e. the guide shaft 111 and the guide rails 112, 113) is fixed to the second translation stage 32 such that, when moving the tank 2 in the x and/or the y direction using the first and/or the second translation stage 31 and/or 32, the position of the detector holder 11 (and thus of the detector mounted on the detector holder 11) relative to the tank 2 will not change. Thus, when carrying out a calibration measurement, both the tank 2 and the detector are moved to a desired location in the x-y plane. After the tank 2 has been moved to the desired x-y position (i.e. horizontal position), measurements can be carried out at different z positions (vertical positions) by moving the detector in the liquid to different locations on the z axis using mechanism 10.

The mechanism 10 for positioning the detector furthers comprise a device (such as a an electric motor 335 connected to the shaft 311, for example, by a semering or a magnetic coupling) for driving the translation shaft 111. It is also conceivable that an actuator (such as a micrometer screw) is provided for (fine) adjustment of the position of the translation shaft 111 and the guide rails 112, 113 (and thus of the detector position) in the x and/or y direction, i.e. the detector holder and thus the detector may be (e.g. manually) moved in the x and/or y direction relative to tank 2.

The measurement device 1 according to the invention may further comprise a controlling device (not shown) communicating, for example, with the translation stages 31, 32, the mechanism 10 for positioning the detector and the detector. The controlling device may be configured in such a way that even if, for example, the translation stages 31, 32, the mechanism 10 for positioning the detector and the detector (and possibly other components of the measurement device such as a software for operating the measurement device and/or for evaluating a detector signal) are not provided by the same manufacturer (and thus use different communication protocols), the controlling device is capable of communicating with the different devices (using e.g. a corresponding protocol command database) as set forth above.

It is possible that a plurality of controllers is provided, for example, an individual controller may be assigned to each one of the translation stages 31, 32 and the mechanism 10 for positioning the detector. It is of course also possible that a single controller operates more than one translation stage. For example, a single controller is configured for operating the translation stages 31, 32 and the mechanism 10 for positioning the detector.

The controller (e.g. a computer or a special hardware) device may be positioned on the patient table along with the other components of the measurement device. It is, however, also possible that the controller (or e.g. also the other components of the measurement device) are arranged on another device used in the operating room.

### Reference signs

- 1: measurement device
- 2: tank
- 3: mechanism for moving the tank
- 10: mechanism for moving the detector
- 11: detector holder
- 21: bottom tank
- 31, 32: translation stage
- 111: translation shaft
- 112, 113: guide rail
- 311: translation shaft of first translation stage
- 312: actuator of first translation stage
- 321: translation shaft of second translation stage
- 322: actuator of second translation stage
- 323: carrier
- 324, 325: lateral guide rail of second translation stage
- 326, 327: slide
- 328: adjustment screw
- 329: guide element
- 330, 331: lateral guide rail of first translation stage
- 332, 333: slide
- 334: base plate
- 3341: adjustment screw

## Claims

1. A measurement device for measuring radiation of a radiation source, comprising
- a container (2) for receiving a liquid;
- a detector for detecting radiation of the radiation source, the detector being arranged in the container (2),
**characterized by**
a mechanism (3) for moving the container (2) to various measurement locations along at least one positioning direction (x, y), wherein the detector is arranged in the container (2) in such a way that it will be moved along with the container (2) when the container (2) is moved in the positioning direction (x, y).

2. The measurement device as claimed in claim 1, further comprising a mechanism (10) for moving the detector relative to the container (2) in a direction (z) essentially perpendicular to the positioning direction (x, y).

3. The measurement device as claimed in claim 2, wherein the mechanism (10) for moving the detector comprises at least guide element (112, 113) to which the detector is mounted, the guide element (112, 113) being moved along with the container (2) when the container (2) is moved in the positioning direction (x, y).

4. The measurement device as claimed in any of the preceding claims, wherein the measurement device (1) is to be arranged in such a way relative to the radiation source that the positioning direction (x, y) extends essentially perpendicular to an axis of the radiation emitted by the radiation source.

5. The measurement device as claimed in any of the preceding claims, wherein the container (2) comprises a bottom (21) and the positioning direction (x, y) extends essentially parallel to the bottom (21) of the container (2).

6. The measurement device as claimed in any of the preceding claims, wherein the mechanism (3) for moving the container (2) is configured for moving the container (2) independently in a first and in a second positioning direction (x, y), the second positioning direction running perpendicular to the first positioning direction.

7. The measurement device as claimed in any of the preceding claims, wherein the mechanism (3) for moving the container (2) comprises a carrier (323) on which the container (2) is mounted.

8. The measurement device as claimed in claim 7, wherein the container (2) and the carrier (323) are integrally formed.

9. The measurement device as claimed in any of the preceding claims, wherein the container (2) is filled with a liquid having a density comparable to that of a human body.

10. The measurement device as claimed in any of the preceding claims, wherein the container (2) has a volume smaller than 150l, smaller than 50l, smaller than 20l or smaller than 10l.

11. The measurement device as claimed in any of the preceding claims, further comprising a controlling device for controlling at least the mechanism (3) for moving the container and the detector.

12. The measurement device as claimed in claim 11, wherein the mechanism (3) for moving the container (2) is controlled via a first protocol and the detector is controlled via a different second protocol, and wherein the controlling device is configured to communicate via both the first and the second protocol.

13. Radiation therapy arrangement, comprising a measurement device (1) as claimed in any of the preceding claims and a patient table, wherein the measurement device (1) is arranged on the patient table.

14. Controlling device for controlling the mechanism for moving the container and the detector of a measurement device (1) as claimed in claims 1 to 10, wherein the mechanism (3) for moving the container is controlled via a first protocol and the detector is controlled via a different second protocol, and wherein the controlling device is configured to communicate via both the first and the second protocol.

15. Method for measuring radiation of a radiation source, comprising the steps of:
- providing a container (2) for receiving a liquid;
- providing a detector for detecting radiation of the radiation source, the detector being arranged in the container (2),
**characterized by**
moving the container (2) in at least one positioning direction (x, y), wherein the detector is arranged in the container (2) in such a way that it is moved along with the container (2) in the positioning direction (x, y).
